# EUROPEAN PATENT APPLICATION

(11) **EP 1 188 424 A1**
(43) Date of publication of application: **20.03.2002**
(21) Application number: 01600018.4
(22) Date of filing: 20.08.2001
(51) Int. Cl.: A61F 2/44, A61F 2/30, A61F 2/46

(54) **Prosthesis for replacement of a whole vertebral unit**

(30) Priority: 21.08.2000 GR 2000100286
(71) Applicant: Kalaitzis, Christos, 54642 Thessaloniki (GR); Theologou, Theologos, 546 42 Salonica, Thessaloniki (GR); Lemaire, Jean Philippe, 21000 Dijon (FR)
(72) Inventor: Kalaitzis, Christos, 54642 Thessaloniki (GR); Theologou, Theologos, 546 42 Salonica, Thessaloniki (GR); Lemaire, Jean Philippe, 21000 Dijon (FR)

(57) **Abstract**

Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31 which has the capability of connection to a posterior fusion system, consists of the front part, which is of octagonal shape as in fig. 1 and special designed screws 31 that can connect it to the posterior fixation pedicle screw system and thus create a complete construction which aims at solving the problem of migration in cases of replacement of a complete vertebral unit.

Hence, we avoid the danger of two independent fusions, which are currently used and are not stable enough when for some reason, usually tumor of the H.S., the laminas, the joints, and the pedicles have been destroyed besides the body. In this case, instability is great and demands minimization of migration's danger. The connection of the posterior pedicle fusion to the prosthesis at the front, and the new way of expanding its length with a toothed mechanism on the two ends where they touch the final plates, ensures quick insertion, and great stability which is necessary in operations of this type.

## Description

### TERMS USED

Pedicle, pedicle screw, lamina, vertebral unit, toothed ratchet.

### TECHNICAL FIELD

Interconnected system for anterior/posterior support of the Human Spine, which replaces the whole vertebral unit, that is the body, the lamina, and the pedicles, which consists of at least 6 or more pedicle screws for posterior support, and telescopically expanding cage for the replacement of the anterior column (vertebral body), which (the cage) carries a connection mechanism on the side of the spinal column, consisting of a notch through which two special designed screws pass, carrying threads on their ends for connection with nut and washer, creating with the other 4 screws and rods a stable construction which allows complete removal and replacement of the vertebral unit and the stabilization of the H.S. mostly in cases where it is demanded, such as in tumors that have destroyed not only the vertebra's body but the posterior elements as well, that is the lamina and the pedicles. Positioning the system requires double approach, firstly posterior in order to remove by resection of the tumor all the other posterior elements that need to be removed, placement of the 6 screws or more via the pedicles and stabilization, and then anterolateral approach for the removal of the body, its replacement with the graft and connection with the two specially threaded end screws of the posterior fusion.

### TECHNICAL LEVEL OF THE PREVIOUS TECHNIQUE.

The level of the previous technique, also requires both anterior and posterior surgical approach and placement of graft or cage at the front combined with posterior fusion with screws. In this type of fixation of the Human Spine and especially in cases of great instability due to destruction of the pedicles, the joints, the lamina etc, it is not easy to ensure great stability and there is danger of failure, even though the two systems cooperate with one another because they are not interconnected. Up until today, in similar operations, various systems are used with which the surgeon replaces the vertebra's body which is removed along with the discs, with telescopically expanding cylinders or others that are cut at an appropriate height, through anterolateral approach, trying to expand the height and then compress the cylinder with plate or rod on the ends of which screws pass into the upper and lower healthy bodies, then at the same or at some other time posterior support with pedicle screws and rods is performed, in an effort to ensure the stability of the anterior cage (CYLINDER) and prevent it from migrating. According to the bibliography, it is necessary to perform posterior support, in order to reduce this danger as much as possible, along with the danger of sinking and penetration of the graft (cylinder) in the healthy bodies, whose endurance to straining we increase with posterior fusion.

### ADVANTAGES OF THE INVENTION

The important new element of our system is the interconnection of the anterior to the posterior system, in such a way, so that there is no case that the cylinder-graft will migrate. This is very important, especially in cases of destruction of the whole vertebral unit, where not only replacement of the anterior section is demanded but also due to removal of the joints, the pedicles, and the lamina there is gap and loss in all the three columns of the H.S., instability, and increased danger of failure. Our system is based on the combination of two different systems, which are not independent one from the other as in previous techniques, but they are firmly connected to one another and eliminate migration danger. The controlled height prosthesis for the anterior replacement of the vertebral body is performed with the aid of springs that sit on teeth. This mechanism is durable to straining, and allows elevation and correction of the vertebral body's height, easily with one distractor without any other tools. Elevation and correction are performed having previously connected the posterior fusion to the anterior graft and then the only task remaining is to fill the gap with autograph or allograft.

### REVELATION

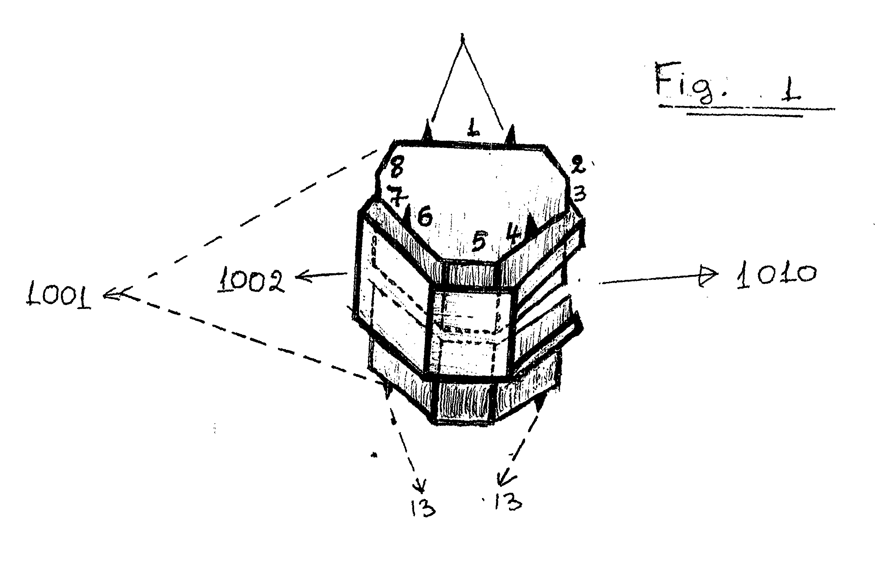

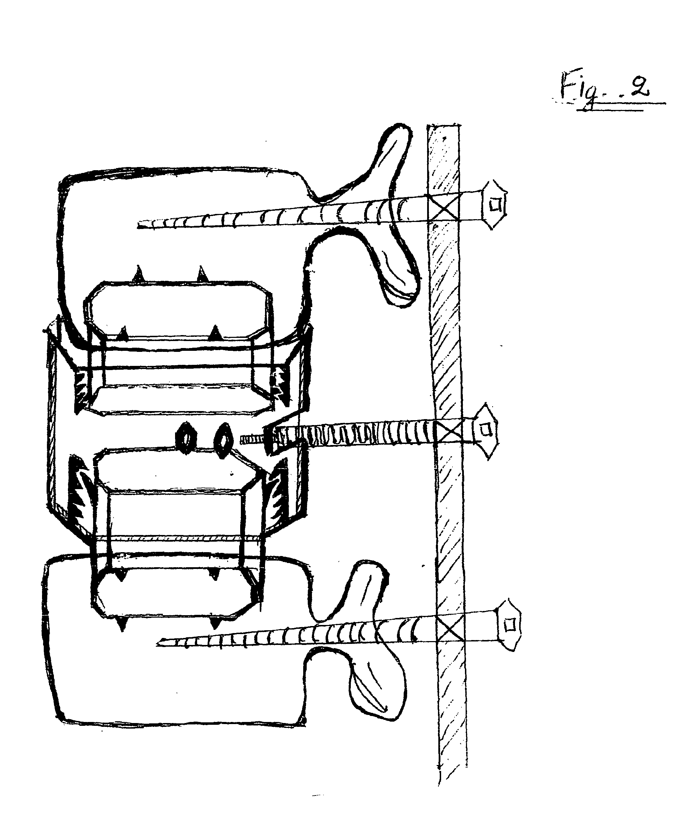

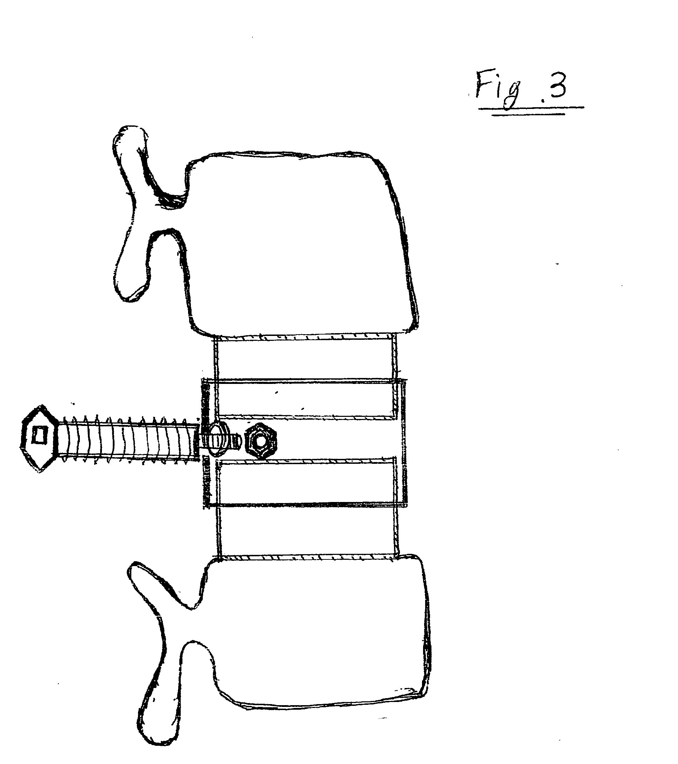

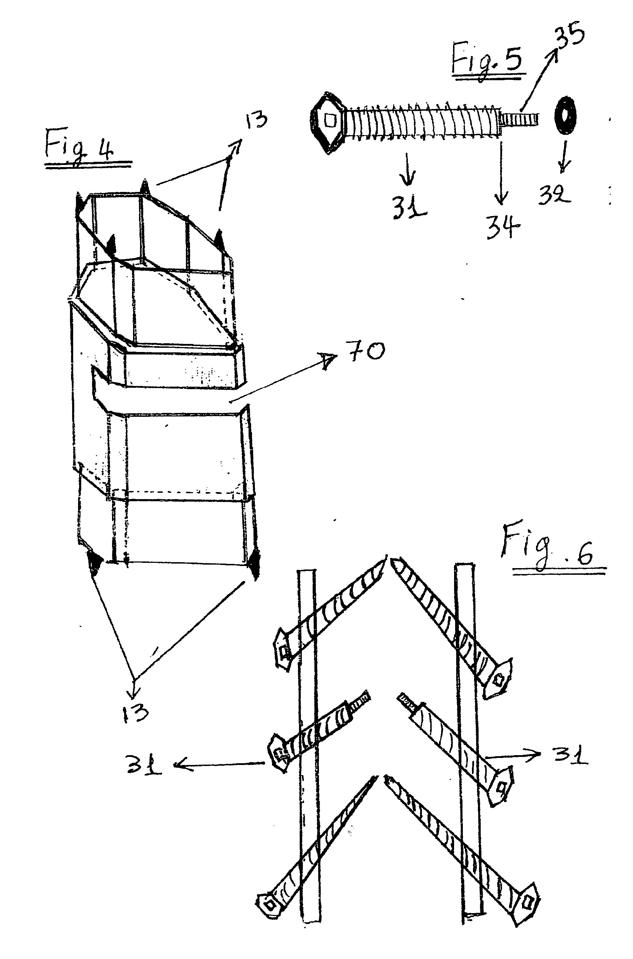

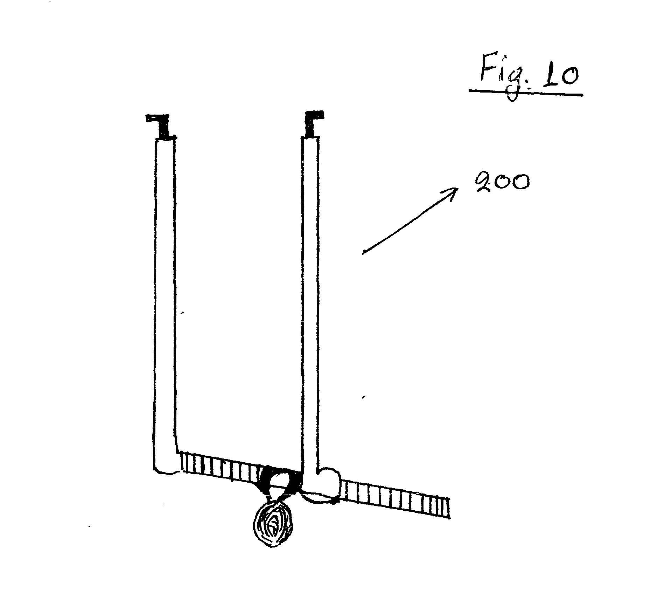

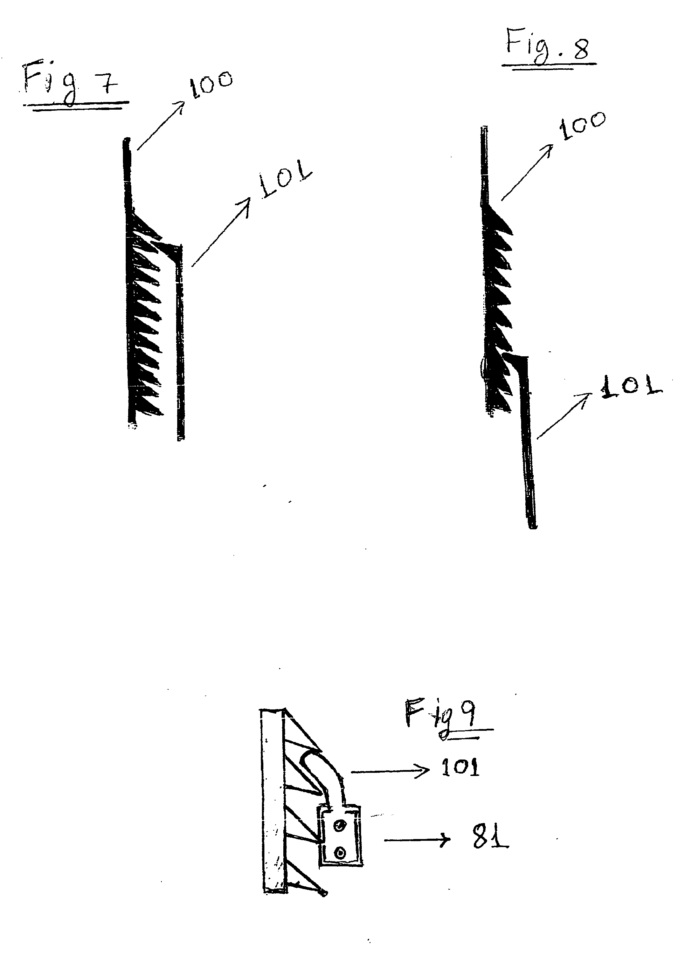

The toothed mechanism 100 for the elevation which is carried in the interior of the prosthesis on both opposite sides 1 and 5 before the expansion is shown on fig. 7, and the mechanism's expansion is shown on fig.8. Fig. 4 illustrates the back side of the prosthesis where notch 70 for the entrance of screws 31 is located. Fig. 6 shows the layout of the pedicle screws' system and the location of screws 31. Fig. 2 and fig. 3 show the way that the posterior and anterior construction are interconnected and secured to one another, and also show the opposite direction of the prosthesis's middle section ratchets, which allow opposite movement. The ratchets may be located on other sides besides 1 and 5 of the octagon, as can be seen on fig. 2. Fig. 1 shows the front part of the prosthesis, and opening 1010 can be seen on the side, onto which the distractor 200 is attached fig. 10 and which also serves as a passage for the graft. The octagon's sides clockwise numbering is also visible. The spring's 101 connection 81 with screws or **pins**, can be seen on fig. 9,on the external section of two opposite sides of the prosthesis's smaller parts and also opposite to the teeth 100, that are located in the internal of the prosthesis's large section, into which the two smaller ones move. Fig. 5 shows the special screw 31 for the interconnection.

The whole system consists of two main sections, the posterior section which is the construction with two rods and at least 6 screws fig. 6, two per vertebra and the prosthesis of the vertebral body fig. 4, for the replacement of the body. These two separate parts are connected to the point where the two of special construction screws 31 with washer 32 and nut 33 are. Screw 31 has a stop 34 onto which the prosthesis rests, so that no backward movement is possible, where the spinal column is, and ends on section 35 which carries threads for nut 33. The prosthesis fig. 1 consists of 3 parts that move close to one another, and is of octagon shape so that it fits to the anatomy of the vertebral body while improving the prosthesis's endurance to straining, the two smaller ones on the ends 1001, which are located in the interior of the bigger one 1002 and that are expanded upwards and downwards with mechanism fig. 7 which consists of spring 101 in the external part of sides 1 and 5 fig. 1 of the two smaller internal parts 1001 and the toothed ratchet 100 that is carried internally by part 1002 into which spring 101 moves and sits in the space between two teeth, the distractor 200 forces the spring to move to the next position every time that distraction force is exercised. In this way, expansion is gradual and controllable, until the desired height is reached. The prosthesis carries a notch 70 on its posterior part, fig. 4, fig. 2, fig.3, which allows the threaded part of the two screws 31 to enter, and then is secured with washer 32 and nut 33 in the interior section of the prosthesis, fig. 2, fig. 3. In this way, the prosthesis which replaces the front part of the body is connected very firmly to the posterior fusion which consists of rods, and screws, allowing not only removal of the vertebral body, but also of the lamina, and the joints, that is of all the vertebral unit, in cases where it is necessary, without the previous techniques' danger.

### EXAMPLE

Patient with destroyed body of the 4^{th} Lumbar vertebra due to cancer, and also sunk and destroyed posterior elements, that is lamina and pedicles, is operated aiming to the removal and cleaning of the tumor and then its support with fusion materials. Incision and uncovering of the posterior elements of L3, L4, and L5 is performed while the patient is put in a supine position, and finally removal of the destroyed joints of L4 and the lamina is performed. Then 4 pedicle screws are inserted via the pedicles of L3 and L5, while in the place where L4's pedicles used to be the special construction screws 31 are inserted, estimating for safety reasons that the stop 34 surpasses the spinal channel and the dura and are connected to the two rods as in fig. 6. Afterwards, having closed the trauma with temporary stitches, we change the patient's position from supine to lateral, so that when we perform retroperitoneal approach we have access to the anterior side of the Human Spine. We remove the cancerous body of L4 with the discs, thus, uncovering the end 35 and the stop 34 of the two screws 31. We then allow notch 70, of the posterior side of the prosthesis fig. 4, slip into the gap which is created. Now, the ends 35 of the screws 31 are positioned in the interior of the prosthesis and we can see them through the opening 1010 of the prosthesis. We put on each one washer 32 and tighten the nut 33. The prosthesis is firmly secured thus being stopped on the one side of the stop 34 and through tightening of the nut on the other. With the distractor's help the ends of which are positioned on opening 1010, we start expanding the two ends 1001 while changing spring's 101 position which moves on teeth 100, while section 1002, is firmly attached on screws 31. Teeth 13 on the upper and lower sections are sunk into the healthy bodies. Expansion of the prosthesis is accomplished with the mechanism's function fig. 7 and fig.8.

## Claims

1. Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31, that pass through notch 70 carried (by the prosthesis) on the back side of its middle and larger dimension's part, from the three which it consists of, which (the prosthesis) is placed closed and is expanded with a distractor's aid, which forces spring 101, of the mechanism fig. 7, fig. 8, fig. 9, to move gradually on the ratchet 100, from one tooth to the next which each time is secured in a new position, allowing the controlled and gradual increase of the prosthesis's length, hence, the increase of the height to the desired point.

2. Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31, which has the capability of being connected to a posterior fusion system as in claim 1, whose notch 70 is located on its back side, that is on side 1 and expands a bit towards sides 2 and 8 of the middle and larger section from the three it consists of.

3. Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31, which has the capability of being connected to a posterior fusion system as in claim 1, whose connection screw 31, has threads 35 and stop 34 onto which the prosthesis stops so that there is no backward movement, on thread 35 passes washer 32 and nut 33 is screwed so that the prosthesis is secured.

4. Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31, which has the capability of being connected to a posterior fusion system as in claim 1, whose toothed mechanism fig. 7, fig. 9, is positioned on two opposite sides, that is side 1 and 5 or 2 and 6, with the spring on sections 1001 externally and the teeth on the interior of 1002 and on the same sides, towards opposite direction upwards and downwards and with such in between space so that when we move parts 1001 with the distractor, they move away from one another along the teeth.

5. Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31, which has the capability of being connected to a posterior fusion system as in claim 1, which carries opening 1010, onto which a distractor can be attached for the expansion of the prosthesis's length and through which graft passes or methacrylic cement.

6. Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31, which has the capability of being connected to a posterior fusion system as in claim 1, which carries teeth 13 on its ends, for firm attachment to the final plates of the healthy vertebral bodies.

7. Prosthesis for the replacement of a complete vertebral unit, whose assembly allows total removal and replacement of all the vertebral body's elements that are the pedicles with the joint facets and the lamina and has interconnection capability to a posterior fusion system which carries special screws 31, which has the capability of being connected to a posterior fusion system as in claim 1, whose end parts can be of conical or flat shape from back to forward so that they are of anatomical shape enabling preservation of the demanded lordosis.
